# EUROPEAN PATENT SPECIFICATION

(11) **EP 2 753 687 B1**
(45) Date of publication and mention of the grant of the patent: **18.04.2018**
(21) Application number: 12780278.3
(22) Date of filing: 10.09.2012
(51) Int. Cl.: C12N 1/20, A61K 35/74, A61K 38/47, A61K 47/44, A61K 31/198, A61K 35/745, A61K 35/747, A23L 33/135, A23L 33/17, A23L 33/175, A23L 33/21

(54) **STRAINS OF LACTIC ACID BACTERIA AND/OR BIFIDOBACTERIA INHIBITING/REDUCING THE GROWTH OF DIFFERENT BIOTYPES OF E. COLI AND DIFFERENT BIOTYPES OF CLOSTRIDIA**
STÄMME VON MILCHSÄUREBAKTERIEN UND/ODER BIFIDOBAKTERIEN ZUR HEMMUNG ODER MINDERUNG DES WACHSTUMS VERSCHIEDENER BIOTYPEN VON E. COLI UND VERSCHIEDENER BIOTYPEN VON CLOSTRIDIEN
SOUCHES DE BACTÉRIES LACTIQUES ET/OU DE BIFIDOBACTÉRIES INHIBANT/RÉDUISANT LA CROISSANCE DE DIFFÉRENTS BIOTYPES DE E. COLI ET DE DIFFÉRENTS BIOTYPES DE CLOSTRIDIA

(30) Priority: 09.09.2011 IT RM20110475
(43) Date of publication of application: 16.07.2014
(73) Proprietor: Probiotical S.p.A., 28100 Novara (NO) (IT)
(72) Inventor: MOGNA, Giovanni, 28100 Novara (NO) (IT)
(74) Representative: Hoffmann Eitle
(86) International application number: PCT/IB2012/001745
(87) International publication number: WO 2013/034975

(56) References cited:
- WO-A1-2010/136891
- WO-A1-2011/017040
- WO-A1-2012/001440
- WO-A2-2007/100765
- US-A1- 2008 175 899
- ZHANG L ET AL: "Evaluation of Lactobacillus rhamnosus GG using an Escherichia coli K88 model of piglet diarrhoea: Effects on diarrhoea incidence, faecal microflora and immune responses", VETERINARY MICROBIOLOGY, ELSEVIER BV, NL, vol. 141, no. 1-2, 24 February 2010 (2010-02-24), pages 142-148, XP026883463, ISSN: 0378-1135, DOI: 10.1016/J.VETMIC.2009.09.003 [retrieved on 2009-09-11]
- K. A. EATON ET AL: "Probiotic Lactobacillus reuteri Ameliorates Disease Due to Enterohemorrhagic Escherichia coli in Germfree Mice", INFECTION AND IMMUNITY, vol. 79, no. 1, 25 October 2010 (2010-10-25), pages 185-191, XP055024810, ISSN: 0019-9567, DOI: 10.1128/IAI.00880-10
- K. C. JOHNSON-HENRY ET AL: "Lactobacillus rhamnosus Strain GG Prevents Enterohemorrhagic Escherichia coli O157:H7-Induced Changes in Epithelial Barrier Function", INFECTION AND IMMUNITY, vol. 76, no. 4, 1 April 2008 (2008-04-01), pages 1340-1348, XP055024814, ISSN: 0019-9567, DOI: 10.1128/IAI.00778-07
- A MARCHESE: "Effect of fosfomycin alone and in combination with N-acetylcysteine on E. coli biofilms", INTERNATIONAL JOURNAL OF ANTIMICROBIAL AGENTS, vol. 22, 1 October 2003 (2003-10-01), pages 95-100, XP055024665, ISSN: 0924-8579, DOI: 10.1016/S0924-8579(03)00232-2
- COLLADO M C ET AL: "Probiotic Strains and Their Combination Inhibit In Vitro Adhesion of Pathogens to Pig Intestinal Mucosa", CURRENT MICROBIOLOGY, SPRINGER-VERLAG, NE, vol. 55, no. 3, 25 July 2007 (2007-07-25), pages 260-265, XP019539746, ISSN: 1432-0991, DOI: 10.1007/S00284-007-0144-8
- DAN YANG YING ET AL: "Microencapsulated Lactobacillus rhamnosus GG Powders: Relationship of Powder Physical Properties to Probiotic Survival during Storage", JOURNAL OF FOOD SCIENCE, vol. 75, no. 9, 1 November 2010 (2010-11-01), pages E588-E595, XP055024657, ISSN: 0022-1147, DOI: 10.1111/j.1750-3841.2010.01838.x
- CHAMPAGNE C P ET AL: "The determination of viable counts in probiotic cultures microencapsulated by spray-coating", FOOD MICROBIOLOGY, ACADEMIC PRESS LTD, LONDON, GB, vol. 27, no. 8, 1 December 2010 (2010-12-01), pages 1104-1111, XP027273852, ISSN: 0740-0020 [retrieved on 2010-07-24]
- HÜTT P ET AL: "Antagonistic activity of probioitic lactobacilli and bifidobacteria aganst entero- and uropathogensal model", JOURNAL OF APPLIED MICROBIOLOGY, vol. 100, no. 6, June 2006 (2006-06), pages 1324-1332, XP002674059, ISSN: 1364-5072
- GUEIMONDE M ET AL: "Adhesion and competitive inhibition and displacement of human enteropathogens by selected lactobacilli", FOOD RESEARCH INTERNATIONAL, ELSEVIER APPLIED SCIENCE, BARKING, GB, vol. 39, no. 4, 1 May 2006 (2006-05-01), pages 467-471, XP025014241, ISSN: 0963-9969, DOI: 10.1016/J.FOODRES.2005.10.003 [retrieved on 2006-05-01]
- MCFARLAND LYNNE V: "Meta-analysis of probiotics for the prevention of antibiotic associated diarrhea and the treatment of Clostridium difficile disease.", THE AMERICAN JOURNAL OF GASTROENTEROLOGY APR 2006 LNKD- PUBMED:16635227, vol. 101, no. 4, April 2006 (2006-04), pages 812-822, XP002674094, ISSN: 0002-9270
- RADA V ET AL: "Susceptibility of bifidobacteria to lysozyme as a possible selection criterion for probiotic bifidobacterial strains", BIOTECHNOLOGY LETTERS, SPRINGER NETHERLANDS, DORDRECHT, vol. 32, no. 3, 27 November 2009 (2009-11-27), pages 451-455, XP019787050, ISSN: 1573-6776
- FERNANDEZ M F ET AL: "Probiotic properties of human lactobacilli strains to be used in the gastrointestinal tract", JOURNAL OF APPLIED MICROBIOLOGY, OXFORD, GB, vol. 94, no. 3, 1 January 2003 (2003-01-01), pages 449-455, XP002283214, ISSN: 1364-5072, DOI: 10.1046/J.1365-2672.2003.01850.X
- Sigrid C.J. De Keersmaecker ET AL: "Strong antimicrobial activity of Lactobacillus rhamnosus GG against Salmonella typhimurium is due to accumulation of lactic acid", FEMS Microbiology Letters, vol. 259, no. 1, 1 June 2006 (2006-06-01), pages 89-96, XP055088376, ISSN: 0378-1097, DOI: 10.1111/j.1574-6968.2006.00250.x

## Description

The present invention refers to strains of lactic bacteria and bifidobacteria, having actions of inhibiting/reducing the growth of different biotypes of *E.coli* (gram-negative), including *E.coli* O157:H7 and O104:H4, and of different bacterial species belonging to the family of the Clostridiaceae (anaerobic, Gram positive rods) responsible for putrefaction and serious intestinal infections (*Clostridium difficile*).
Furthermore, the present invention refers to strains of lactic bacteria having specific antimicrobial activity against *Listeria monocytogenes* (gram-positive), *Enterococcus sp.* and *Klebsiella sp.* (gram-negative). Furthermore, the present invention refers to a pharmaceutical or dietary composition or a supplement or a medical device comprising at least one of said strains of lactic bacteria and/or bifidobacteria, optionally in combination with acetylcysteine and/or lysozyme.
It is known that *Escherichia coli -* abbreviated to *E. coli -* is the best-known species of the genus *Escherichia*: within it, at least 171 serotypes are distinguished, characterised by different combinations of the antigens O, H, K, F. It is one of the principal species of bacteria which live in the lower part of the intestine of warm-blooded animals (birds and mammals, including man), and which are necessary for the correct digestion of food. Its presence in bodies of water indicates the presence of faecalisation conditions (it is the principal indicator of faecal contamination, together with the enterococci).
The genus *Escherichia,* together with other genera such as for example Enterobacter, Klebsiella, Citrobacter and Serratia, are grouped together under the name coliforms. Technically the "coliform group" comprises non-sporogenous aerobic and anaerobic bacteria.
Even though it is a common inhabitant of the intestine and has a fundamental role in the digestive process, there are situations in which *E. coli* can cause illnesses in man and animals. Some strains of *E. coli* are the causative agent of intestinal and extra-intestinal illnesses such as infections of the urinary tract, meningitis, peritonitis, septicaemia and pulmonitis. Some strains of *E. coli* are toxigenic, i.e. they produce toxins which can be the cause of diarrhoea among other things. Dysentery caused by *E. coli* is a common alimentary toxinfection, since it is contracted principally from contaminated foodstuffs. Contamination can occur from inadequately-cooked infected meat, from unpasteurised milk and cheeses made from it, and from other foodstuffs contaminated by faeces. *E. coli* produces four types of toxins which are distinguished, by their different sensitivity to thermal treatment, into thermolabile and thermostable, and by their toxigenic action (Shiga toxins and haemolytic toxins, HlyA).
The thermolabile toxin, named LT, is very similar in structure and functions to the cholera toxin. It contains an 'A' subunit and five 'B' subunits in a holotoxin. The B subunits contribute to adherence and to the toxin's entry into the intestinal cells of the host, where the A subunit stimulates the cells to release water, causing diarrhoea.
A "strain" of *E. coli* is a group with particular characteristics which are able to make it recognisable by other strains of *E. coli*, similarly to the way animals of different breeds can be distinguished. Different strains of *E. coli* live in different animal species, so that it is possible to establish whether faecal material in water comes, for example, from human beings or birds.
New strains of *E. coli* arise continually from the natural biological process of mutation, and some of these strains have characteristics which may be harmful to a host animal. Although in the majority of adult humans a pathogenic strain would probably not cause anything but diarrhoea, and could not produce any symptoms, in small children or sick people or those debilitated by recent illnesses or undergoing particular treatments, a new strain could cause serious illnesses and even death. An example of a particularly virulent strain of *E. coli* is *E.coli* O157:H7. The strains of enterohaemorrhagic *E. coli* are the principal ones responsible for illness in industrialised countries. The serotype principally responsible for these illnesses is *E.coli* O157:H7.
*E. coli* O157: H7 or enterohaemorrhagic *Escherichia coli* (EHEC) is an enterohaemorrhagic strain of the bacterium *Escherichia coli* which is a cause of diseases transmitted by food. Infection often leads to haemorrhagic diarrhoea and, occasionally, to renal failure, haemolytic-uraemic syndrome (HUS), especially in young people, children and old people.
Transmission occurs by the faecal-oral route, and the majority of cases of illness are associated with dietary consumption of raw or under-cooked foods, contaminated by the soil or contaminated water, or consumption of vegetables polluted by such water. In ruminants it lives as a commensal in the intestine, but does not give rise to pathology because of the scarcity of receptors for the toxin produced by the bacterium. With reference to the genus *Clostridium,* it is known that many foods contain these bacteria which are responsible for the degradation processes of the foods themselves, and that if they are ingested with the diet and reach the intestine, they are able to modify the composition of the microflora and initiate putrefaction.
The foods generally responsible for these alimentary toxinfections are fresh meats contaminated in particular by Clostridium perfringens, C. sporogenes, C. bifermentans, etc.), which can hydrolyse sugars forming malodorous substances such as indole, ammonia and mercaptans.
Fish, on the other hand, can be attacked by another species of *Clostridium, C. botulinum* which can develop and produce a toxin even at low temperatures (4°C). Among the Clostridia most dangerous to man is *Clostridium difficile,* which can produce enterotoxins responsible for cases of haemorrhagic diarrhoea, pseudomembranous colitis with serious complications such as perforation, septicaemia and death. The bacterium forms spores resistant to heat and is transmitted from person to person via the faecal-oral route.
Generally the pathogenesis caused by *C. difficile* is associated with the use of antibiotics which cause marked imbalance in the intestinal microflora, causing the prevalence of putrefacient species, to the detriment of those with fermentative action.
*C. difficile* is ubiquitous, in particular in hospital environments, and can colonise the intestine of a small percentage of healthy individuals (about 5%). The use of antibiotics is associated with its excessive development and with its serious pathological manifestations due to its resistance to the majority of antibiotics. The spores ingested, once they have overcome the gastric barrier, settle in the colon where they germinate and evolve into the vegetative form, which by binary multiplication generates a numerous population responsible for the multiple pathological forms which are particularly serious in the elderly.

It is estimated that currently this organism infects more than 30% of patients treated in hospitals in the United States. In addition, *Listeria monocytogenes* is a ubiquitous pathogen responsible in man for listeriosis, a potentially fatal infection of alimentary origin.
Probiotic bacteria (lactobacilli and bifidobacteria) are micro-organisms which benefit the health of the host, and for this reason are commonly consumed by an ever-increasing number of people who choose food products, fermented milk products, yogurt and supplements containing probiotic bacteria.

Zhang et al (Veterinary microbiology Elsevier BV, NL, vol. 141, no. 1-2, 24 February 2010, pages 142-148) deals with the evaluation of Lactobacillus rhamnosus GC, in particular the specific activity against one single E.coli strain, K88.

Also K. A. Eaton et al (Infection and Immunity, vol. 79, no. 1, 25 October 2010, pages 185-191) deals with the activity of a lactic acid bacterium against a single bacterial strain, EHEC strain 86-24 (see e.g. page 186, Materials and Methods).

Also Johnson-Henry et al (Infection and Immunity, vol. 76, no. 4, 1 April 2008, 1340-1348) deals with the activity of a bacterial strain against one single E. coli strain (see e.g. page 1341, left column, third paragraph).

US 2008/175899 relates to a formulation for promoting sinus health includes at least one probiotic agent in combination with N-acetyl cysteine (NAC) and a bioflavonoid or derivative thereof.

Marchese (International Journal of Antimicrobial Agents vol. 22, 1 October 2003, 95-110) relates to the effects of fosfomycin, alone and in combination with N-acetyl cysteine, on E. coli biofilms.

WO 2011/017040 is relative to a nutritional composition including a fiber blend having agglomerated fiber particulates and a probiotic.

Rada et al (Biotechnology Letters, vol. 32, no. 3, 27 November 2009, 451-455) deals with the susceptibility of bifidobacteria to lysozyme as a possible selection criterion for probiotic bifidobacterial strains.

WO 2007/100765 relates to methods and compositions for treating sepsis in a subject in need thereof, the method comprising the administration of effective amounts of at least one lysozyme-modified probiotic component.
WO 2012/001440 relates to a composition comprising two probiotic strains of bacteria capable of performing an antioxidant and/or antibacterial and competitive action against species of coliform bacteria that produce gas by fermentation of sugar, in particular lactose, isolated from infants affected by colic.
WO 2010/136891 A1 is relative to the use of *L. pentosus* LPS 01 (DSM 21980) for the treatment of infections from Gram-negative bacteria, including *E. coli.*

There remains a need, therefore, to have available probiotic bacteria capable of colonising the intestine and bringing beneficial effects to the host. In particular, there remains a need to have available probiotic bacteria capable of prevailing over pathogenic species, with consequent health benefits. Even more particularly, there remains a need to have available probiotic bacteria capable of preventing and/or treating infections and/or pathologies connected with pathogenic bacteria (gram-positive and gram-negative).
Following intensive research activity, the Applicant has surprisingly found that selected bacteria are capable of giving an appropriate response to the above-mentioned needs.
The Applicant has, furthermore, surprisingly found that by using N-acetylcysteine, which has the characteristic of dissolving the bacterial biofilms normally produced in self-defence by the pathogenic germs, the bacteriocin action of the selected probiotic strains is much more effective.
It has furthermore surprisingly found that by using a particular technique of microencapsulation in a lipid matrix, preferably of a vegetable nature, which confers gastroprotection on the lysozyme, the latter is enabled to pass undamaged through the gastroduodenal tract, to be activated at colon level and to express its lytic action against the spores of all species of clostridia. As a consequence of this hydrolytic action on the spores of the genus *Clostridium* and *C. difficile* in particular, at the moment of their germination, the formation of vegetative cells responsible for the increase in the pathogen population and the formation of toxins does not occur.
The combined action of probiotic strains with fermentative activity and barrier effect against the putrefacient species, including the clostridia, together with the direct action of the lysozyme which in fact prevents the germination of the clostridia spores, constitutes an extraordinary and innovative strategy against this dangerous group of pathogens.
The total combination (probiotic bacteria of the present invention producing bacteriocins, and/or N-acetylcysteine which dissolves the bacterial biofilm, and/or lysozyme, preferably in microencapsulated form in a lipid matrix, which blocks the germination of the spores) constitutes an innovative tool capable of combating a large number of pathogenic strains, against which up to now there was no. effective strategy of opposition.
A subject of the present invention is formed by a selection of strains of lactic bacteria which are capable of producing bacteriocins and/or metabolites and/or hydrogen peroxide (oxygenated water) for use in the preventive and/or curative treatment of infections and/or pathologies connected with: the various pathogens, including *E. coli* and in particular *E. coli* O157:H7 and O104:H4; and/or pathogens *Listeria monocytogenes, Enterococcus sp.,* said bacteria being also, optionaly, used in combination with N-acetylcysteine and/or lysozyme. The lysozyme can be microencapsulated and gastroprotected in a lipid matrix. In a preferred embodiment, the lipid matrix is of vegetable origin having a melting point comprised from 30°C to 80°C, preferably from 40°C to 70°C, even more preferably from 50°C to 60°.

Another subject of the present invention is formed by a pharmaceutical or dietary composition or a supplement or a medical device comprising at least one of the strains of bacteria according to claim 1 for use in the preventive and/or curative treatment of infections and/or pathologies connected with *E. coli* pathogens, including *E. coli* O157:H7; and/or pathogens *Listeria monocytogenes, Enterococcus sp.*
Another subject of the present invention is formed by a pharmaceutical or dietary composition or a supplement or a medical device comprising at least one of the said strains of bacteria in combination with N-acetylcysteine.
Another subject of the present invention is formed by a pharmaceutical or dietary composition or a supplement or a medical device comprising at least one of the said strains of bacteria in combination with lysozyme. In a preferred embodiment, the lysozyme is microencapsulated.
Another subject of the present invention is formed by a pharmaceutical or dietary composition or a supplement or a medical device comprising at least one of the said strains of bacteria in combination with N-acetylcysteine and with lysozyme. In a preferred embodiment, the lysozyme is microencapsulated with a lipid matrix. In a preferred embodiment, the lipid matrix is of vegetable origin having a melting point comprised from 30°C to 80°C, preferably from 40°C to 70°C, even more preferably from 50°C to 60°.
Other preferred embodiments of the present invention are described in the continuation of the description and will be claimed in the attached dependent claims.

### List of Tables and Drawings

Table 1 shows a list of 14 strains of lactobacilli, 4 strains of bifidobacteria and 2 strains of streptococci which have been tested against 4 biotypes of *E coli.*
Tables 2-5 list the strains which produce bacteriocins and/or metabolites and/or oxygenated water and which are able to combat, inhibit or reduce the growth of pathogenic bacteria belonging to the species *E. coli*, including *E. coli* O157:H7.
Tables 6 and 7 refer to the strains of the genus *Lactobacillus.*
Figure 1 shows the technique used for testing the strains which are the subject of the present invention.
Figure 2 shows the inhibition halos formed by the strains of Table 4, against *E. coli* (ATCC 8739 ATCC 10536 ATCC 35218 ATCC 25922) and serotype O157:H7 (CQ 9485).

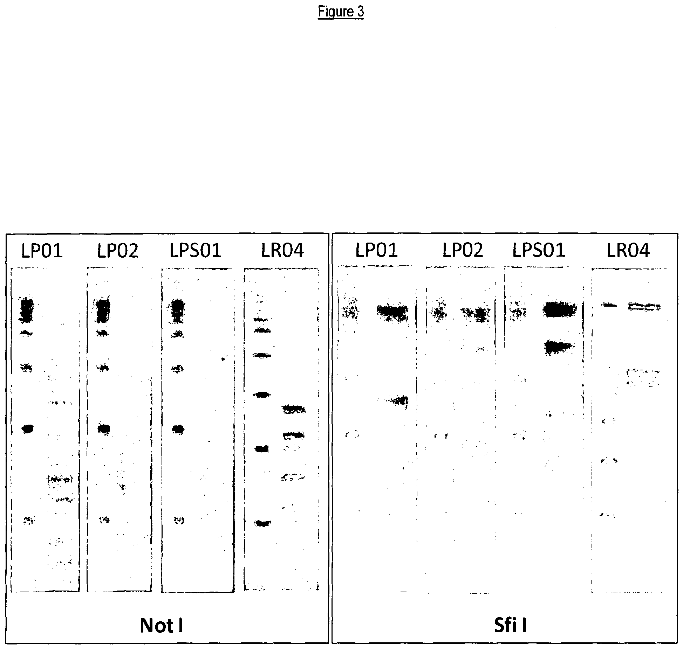

Figure 3 refers to the data shown in Tables 6 and 7.
Figure 4 refers to the *in vitro* inhibitory activity of the strains of probiotic bacteria against *E.coli.*

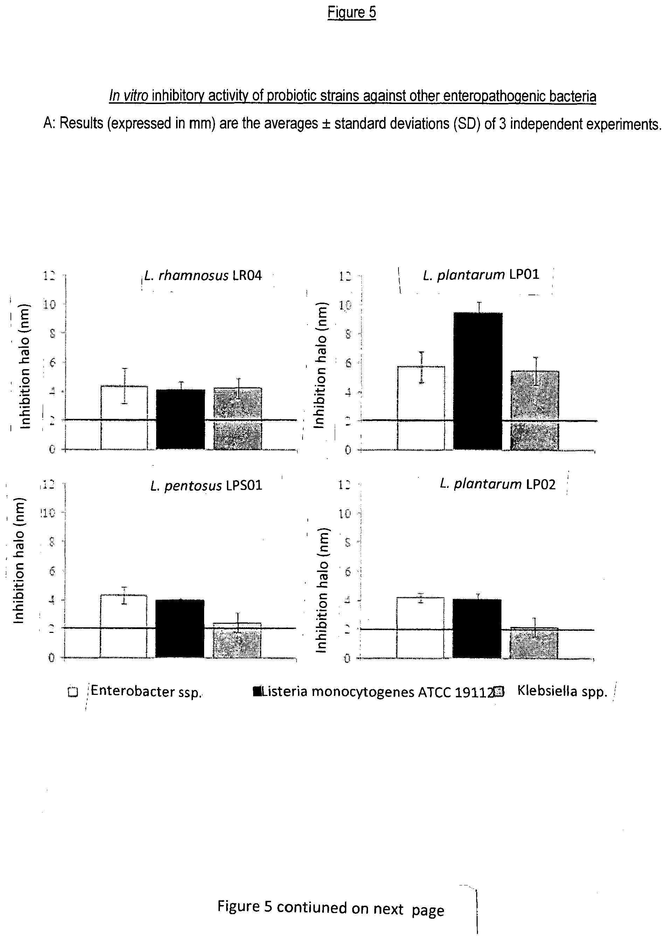

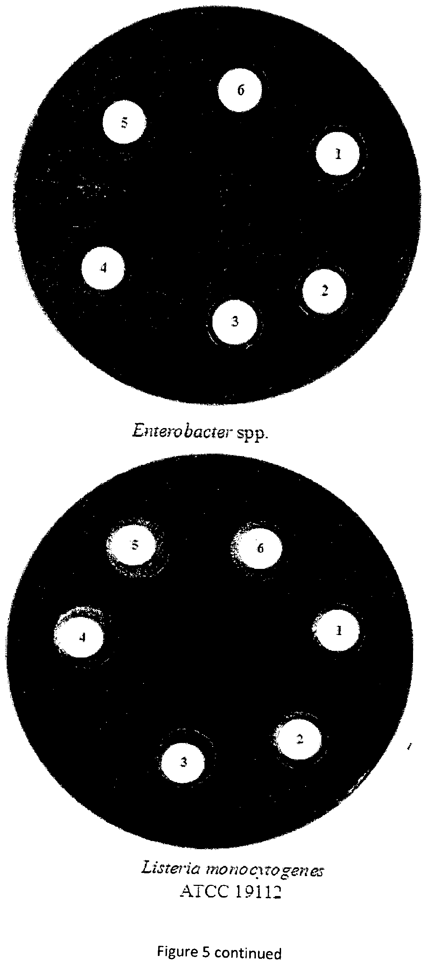

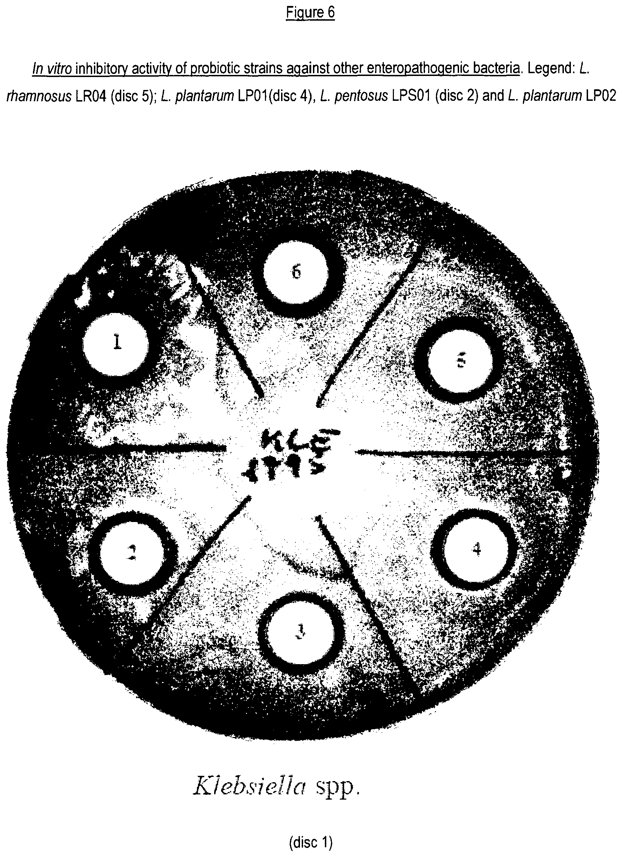

Figures 5 and 6 refer to the *in vitro* inhibitory activity of the strains of probiotic bacteria against other enteropathogenic bacteria.

The Applicant has conducted intense research and screening activity starting from a very extensive group of strains of bacteria. At the end of this activity, the Applicant found and selected a group of strains of bacteria belonging to a species or to several species selected from the group comprising or, alternatively, consisting of *Lactobacillus plantarum, Lactobacillus rhamnosus* and *Lactobacillus pentosus.* The selected strains are capable of producing bacteriocins and/or metabolites and/or oxygenated water, these being substances which are capable of effectively combating, inhibiting or reducing pathogenic bacteria. These strains have a valid application and use in the preventive and/or curative treatment of infections and/or pathologies connected with gram-negative and/or gram-positive pathogenic bacteria.

The pathogenic bacteria are selected from the group comprising the coliforms and clostridia. The coliforms are a group of bacteria belonging to the family of *Enterobacteriaceae.* In particular, the coliforms are rod-shaped, gram-negative, asporogenous, aerobic and anaerobic facultative bacteria, which ferment lactose, with the production of gas and acids, at 35-37°C in 48 hours and possess the enzyme beta-galactosidase. They are ubiquitous organisms; some are present in faecal material, and are therefore used as indicators of pollution both of water and of food, others are of aquatic or telluric origin. The group comprises more than fifty genera, among them *Citrobacter, Enterobacter,* preferably *Enterobacter cloacae, Escherichia,* preferably *E. coli*, *Hafnia, Klebsiella,* preferably *Klebsiella pneumoniae, Serratia* and *Yersinia.* Other pathogens always of interest in the context of the present invention belong to the family of the Clostridiaceae, with particular reference to *C. difficile.* Antagonistic action is exerted also against species selected from the group comprising *Salmonella enteriditis, Campylobacter jejunii, Helicobacter pylori, Listeria monocytogenes, Enterococcus sp.* and *Klebsiella sp.*

A subject of the present invention is formed by a pharmaceutical or dietary composition or a supplement or a medical device comprising at least one bacterial strain as recited in claim 1 belonging to a species or several species selected from the group comprising or, alternatively, consisting of: *Lactobacillus plantarum, Lactobacillus rhamnosus, Lactobacillus pentosus, ,* capable of producing bacteriocins and/or metabolites and/or oxygenated water for use in the preventive and/or curative treatment of infections and/or pathologies connected with pathogens such as *E. coli*, *Listeria monocytogenes* and *Enterococcus sp.*

In one embodiment, the pharmaceutical or dietary composition or supplement or the medical device comprises at least one bacterial strain as recited in claim 1 belonging to a species or several species selected from the group comprising or, alternatively, consisting of: *Lactobacillus plantarum* and *Lactobacillus rhamnosus,* capable of producing bacteriocins and/or metabolites and/or oxygenated water for use in the preventive and/or curative treatment of infections and/or pathologies connected with pathogens such as *E. coli*, *Listeria monocytogenes,* and *Enterococcus sp.*

The strain and/or the various possible combinations of the above-mentioned strains (see also Tables 1-5 below) selected for said purpose may be used alone or in combination with N-acetylcysteine; or in combination with microencapsulated lysozyme; or in combination with N-acetylcysteine and microencapsulated lysozyme.

In one embodiment, the pathogen *E. coli* is selected from among *E. coli* O157:H7 and *E. coli* O104:H4.

Table 1 shows a list of 14 strains of lactobacilli, 4 strains of bifidobacteria and 2 strains of streptococci which have been tested against 4 biotypes of *E coli.* The values are expressed as inhibition halo (mm).

**Table 1**

| | | | **Antimicrobial activity against *E. coli*** | | | |
|---|---|---|---|---|---|---|
| **Species** | **Strain** | **Filing No.** | **ATCC 8739** | **ATCC 10536** | **ATCC 35218** | **ATCC 25922** |
| *B. animalis subsp. lactis* | BS01 | LMG P-21384 | + | + | - | - |
| *B. breve* | BR03 | DSM 16604 | ++ | ++ | + | + |
| *B. breve* | B632 | DSM 24706 | ++ | ++ | + | + |
| *B. longum* | BL05 | DSM 23234 | - | - | - | - |
| *L. paracasei* | LPC00 | LMG P-21380 | - | - | - | - |
| *L. paracasei* | LPC08 | DSM 21718 | - | - | - | - |
| *L. rhamnosus* | LR04 | DSM 16605 | ++ | ++ | ++ | +++ |
| *L. rhamnosus* | LGG | ATCC 53103 | - | + | - | - |
| *L. rhamnosus* | LR06 | DSM 21981 | ++ | ++ | + | + |
| *L. fermentum* | LF10 | DSM 19187 | - | - | - | - |
| *L. plantarum* | LP01 | LMG P-21021 | ++ | ++ | ++ | + |
| *L. plantarum* | LP02 | LMG P-21020 | + | ++ | + | + |
| *L. plantarum* | LP03 | LMG P-21022 | ++ | +++ | - | + |
| *L. plantarum* | LP04 | LMG P-21023 | ++ | +++ | - | + |
| *L. pentosus* | LPS01 | DSM 21980 | ++ | ++ | +++ | +++ |
| *L. delbr. subsp. delbrueckii* | LDD01 | DSM 22106 | ++ | +++ | ++ | +++ |
| *L. reuteri* | LRE01 | DSM 23877 | + | + | - | - |
| *L. reuteri* | LRE03 | DSM 23879 | + | + | - | - |
| *S. thermophilus* | YO2 | DSM 16590 | - | - | - | - |
| *S. thermophilus* | YO3 | DSM 16591 | - | - | - | - |

The bacteria tested in Table 1, taken singly or in combination with each other, have a valid application in the context of the present invention. The strains of bacteria are selected from the group comprising or, alternatively, consisting of those listed in Table 2.

**Table 2**

| **Species** | **Strain** | **Filing No.** |
|---|---|---|
| *B. breve* | BR03 | DSM 16604 |
| *B. breve* | B632 | DSM 24706 |
| *L. rhamnosus* | LR04 | DSM 16605 |
| *L. rhamnosus* | LR06 | DSM 21981 |
| *L. plantarum* | LP01 | LMG P-21021 |
| *L. plantarum* | LP02 | LMG P-21020 |
| *L. plantarum* | LP03 | LMG P-21022 |
| *I plantanim* | LP04 | LMG P-21023 |
| *L. plantarum* | LP04 | LMG P-21023 |
| *L. pentosus* | LPS01 | DSM 21980 |
| *L. delbr. subsp. Delbrueckii* | LDD01 | DSM 22106 |
| *L. reuteri* | LRE01 | DSM 23877 |
| *I reuteri* | LRF03 | DSM 23879 |
| *L. reuteri* | LRE03 | DSM 23879 |
| *L. reuteri* | LRE03 | DSM 23879 |

The strains of Table 2 are capable of producing bacteriocins and/or metabolites and/or oxygenated water which are able to combat, inhibit or reduce the growth of pathogenic bacteria belonging to the species *E. coli*, including *E. coli* O157:H7.

The strains of Table 2 have been individually tested for the purpose of identifying their capacity for antagonising (inhibiting the growth or reducing the number of one or more harmful or pathogenic microbial species/genera), as stated in the four columns on the right in Table 1.

All the strains described and/or claimed in the present patent application have been deposited in accordance with the Treaty of Budapest and are put at the disposal of the public on request to the competent Depositing Authority. The strains of bacteria are selected from the group comprising or, alternatively, consisting of those listed in Table 3.

**Table 3**

| **Species** | **Strain** | **Filing No.** |
|---|---|---|
| *B. breve* | BR03 | DSM 16604 |
| *B. breve* | B632 | DSM 24706 |
| *L. rhamnosus* | LR04 | DSM 16605 |
| *L. rhamnosus* | LR06 | DSM 21981 |
| *L. planfarum* | LP01 | LMG P-21021 |
| *L. plantarum* | LP02 | LMG P-21020 |
| *L. plantarum* | LP03 | LMG P-21022 |
| *L. plantarum* | LP04 | LMG P-21023 |
| *L. plantarum* | LP04 | LMG P-21023 |
| *L. pentosus* | LPS01 | DSM 21980 |
| *L. delbr. subsp. Delbrueckii* | LDD01 | DSM 22106 |

The strains of bacteria are selected from the group comprising or, alternatively, consisting of those listed in Table 4.

**Table 4**

| **Species** | **Strain** | **Filing No.** |
|---|---|---|
| *B. breve* | BR03 | DSM 16604 |
| *B. breve* | B632 | DSM 24706 |
| *L. rhamnosus* | LR04 | DSM 16605 |
| *L. rhamnosus* | LR06 | DSM 21981 |
| *L. plantarum* | LP01 | LMG P-21021 |
| *L. plantarum* | LP02 | LMG P-21020 |
| *L. pentosus* | LPS01 | DSM 21980 |
| *L. delbr. subsp. Delbrueckii* | LDD01 | DSM 22106 |

The composition of the present invention has further valid application also for use in the preventive and/or curative treatment of infections and/or pathologies connected with the pathogens *E. coli*, *H. pylori* and *Clostridium difficile.*

In one embodiment, the composition of the present disclosure comprises or, alternatively, consists of from one to eight strains, selected from the group comprising or, alternatively, consisting of those listed in Table 4. The composition of the present disclosure comprises or, alternatively, consists of from one to six strains, preferably of from two to five strains, even more preferably of three or four strains, selected from the group comprising or, alternatively, consisting of the strains listed in Table 4. The composition comprises the six strains, as listed in Table 5; or of the 4 lactobacilli of Table

**Table 5**

| **Species** | **Strain** | **Filing No.** |
|---|---|---|
| *B. breve* | BR03 | DSM 16604 |
| *B. breve* | B632 | DSM 24706 |
| *L. rhamnosus* | LR06 | DSM 21981 |
| *L. plantarum* | LP01 | LMG P-21021 |
| *L. pentosus* | LPS01 | DSM 21980 |
| *L. delbr. subsp. Delbrueckii* | LDD01 | DSM 22106 |

The Applicant has found that the strains of bacteria selected from the group comprising or, alternatively, consisting of *L. pentosus* LPS01 DSM 21980, *L. plantarum* LP01 LMG P-21021, *L. plantarum* LP02 LMG P-21020 and *L. rhamnosus* LR04 DSM 16605 are able to exert an inhibitory activity against the 4 strains of *E. coli*; in particular the strains *L. rhamnosus* LR04 DSM 16605 and *L. pentosus* LPS01 DSM 21980 show greater activity also against the serotype O157:H7.

Furthermore, the Applicant has found that the strain *L. plantarum* LP01 LMG P-21021 showed inhibitory capacity against all the enteropathogenic bacteria, in particular against *Listeria monocytogenes.*

A subject of the present disclosure is formed by a pharmaceutical or dietary composition or a supplement or a medical device comprising at least one bacterial strain belonging to a species or several species selected from the group comprising or, alternatively, consisting of: *Lactobacillus plantarum, Lactobacillus rhamnosus* and *Lactobacillus pentosus* which is capable of producing bacteriocins and/or metabolites and/or oxygenated water for use in the preventive and/or curative treatment of infections and/or pathologies connected with pathogens such as *E. coli*, including serotype O157:H7, and *Listeria monocytogenes.*

In one embodiment, said pharmaceutical or dietary composition or supplement or medical device comprises at least one bacterial strain selected from the group comprising or, alternatively, consisting of *L. plantarum* LP01 LMG P-21021 and *L. rhamnosus* LR04 DSM 16605; advantageously it comprises or consists of the strains *L. rhamnosus* LR04 DSM 16605; or it comprises or consists of the strain *L. plantarum* LP01 LMG P-21021.

In the composition of the present invention, the mixture of strains of bacteria can be present in a quantity comprised between 0.5% and 20% by weight, compared with the total weight of the composition, preferably between 2.5% and 10%. The composition can furthermore comprise at least one prebiotic fibre and/or carbohydrates with bifidogenic action.

The prebiotic fibre which has application in the composition of the present invention is a fibre which must be used by the strains of bacteria present in the composition, but not by the pathogens which it is intended to antagonise.

Furthermore, the composition can also comprise other active ingredients and/or components such as vitamins, minerals, bioactive peptides, substances with anti-oxidising, hypocholesterolaemic, hypoglycaemic and anti-inflammatory action and anti-sweetening agents in a quantity generally comprised between 0.001% and 20% by weight, preferably between 0.01% and 5% by weight, always depending on the type of active component and its recommended daily dose if any, compared with the total weight of the composition.

The dietary composition which is the subject of the present invention, for example a symbiotic composition, or a supplement or a pharmaceutical composition or a medical device, is prepared according to the techniques and the equipment known to experts in the field. The composition can contain bacteria in a concentration comprised between 1x10⁶ and 1x10¹¹ CFU/g of mixture, preferably between 1x10⁸ and 1x10¹⁰ CFU/g of mixture. The composition can contain bacteria in a concentration comprised between 1x10⁶ and 1x10¹¹ CFU/dose, preferably between 1x10⁸ and 1x10¹⁰ CFU/dose.

The dose can be comprised between 0.2 and 10 g, for example it is of 0.25 g, 1 g, 3 g, 5 g or 7 g.

The probiotic bacteria used in the present invention can be in solid form, in particular in the form of powder, dehydrated powder or lyophilized.

All the compositions of the present invention are prepared according to techniques known to experts in the field, and by the use of known equipment.

In a preferred embodiment, the composition which is the subject of the present invention comprises furthermore acetylcysteine (or N-acetylcysteine) which is an N-acetylated derivative of the amino acid cysteine, and lysozyme, preferably microencapsulated and gastroprotected in a lipid matrix, as described above.

The Applicant has found that the use of acetylcysteine in association with one or two of the strains of bacteria, described in Tables 1-5, or in the various preferred embodiments mentioned above, is capable of dissolving the bacterial biofilm produced by the pathogenic bacteria themselves and which is used by said pathogens as protection. In practice it has been seen that the pathogenic bacteria are capable of forming a protective coating (biofilm) around the cells. The biofilm makes the cells of the pathogens more difficult to attack and better protected. Acetylcysteine is capable of penetrating the biofilm of the cells and dissolving it, facilitating the attack on the pathogenic cells by means of the bacteriocins and/or the metabolites and/or the oxygenated water produced by the strains of bacteria which are the subject of the present invention.

The quantity of acetylcysteine present in the composition to be administered, which is the subject of the present invention, is comprised between 10 and 1,000 mg/day, preferably it is comprised between 200 and 600 mg/day.

Acetylcysteine, preferably in solid form, is mixed with the probiotic bacteria, preferably in solid or lyophilised form, using techniques and equipment known to experts in the field.

The quantity of lysozyme, preferably microencapsulated, present in the composition to be administered, which is the subject of the present disclosure, is comprised between 10 and 2.000 mg/day, preferably it is comprised between 400 and 1.000 mg/day. The lysozyme (preferably microencapsulated) is preferably in solid form and is mixed with the probiotic bacteria, preferably in solid or lyophilised form, using techniques and equipment known to experts in the field.

The quantities of acetylcysteine and microencapsulated lysozyme present in the composition to be administered, which is the subject of the present disclosure, are respectively comprised between 10 and 1,000 mg/day, preferably between 200 and 600 mg/day, while the microencapsulated lysozyme is comprised between 10 and 2,000 mg/day, preferably between 400 and 1,000 mg/day; they are mixed with the probiotic bacteria in solid form, preferably in solid or lyophilised form, using techniques and equipment known to experts in the field. The quantity of N-acetylcysteine present in the composition which is the subject of the present disclosure is comprised between 10 and 1,000 mg/day, preferably between 50 and 200 mg/day, even more preferably between 60 and 150 mg/day. N-acetylcysteine is available on the market in a pharmaceutically acceptable form, preferably in solid form, and is mixed with the probiotic bacteria, preferably in solid or lyophilised form, using techniques and equipment known to experts in the field to give a homogeneous composition.

The quantity of lysozyme, preferably microencapsulated and gastroprotected, present in the composition which is the subject of the present disclosure, is comprised between 10 and 2,000 mg/day, preferably it is comprised between 400 and 1,000 mg/day, even more preferably between 500 and 800 mg/day, preferably in solid form; it is mixed with the probiotic bacteria, preferably in solid or lyophilised form, using techniques and equipment known to experts in the field to give a homogeneous composition. Lysozyme is available on the market in a pharmaceutically acceptable form.

### Experimental section

The antimicrobial activity of 14 strains of lactobacilli, 4 strains of bifidobacteria and 2 strains of streptococci was tested against 4 biotypes of *E coli.* ATCC 8739, ATCC 10536, ATCC 35218 and ATCC 25922; these are available to the public.

The values are expressed as inhibition halo (mm). The data are shown in Table 1.

To determine the antimicrobial activity, the strains were tested following the protocol set out in Santini et al. (2010) Characterization of probiotic strains: an application as feed additives in poultry against Campylobacter jejuni. Int J Food Microbiol. 2010 Jul 31;141 Suppl 1:S98-108. Epub 2010 Apr 8.

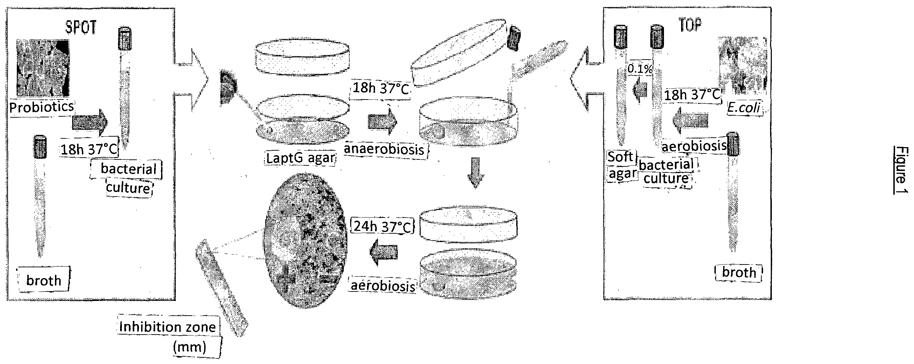

The strains of bacteria listed in Table 1 were cultivated in an MRS broth at 37°C for 18 hours using a system for generating an anaerobic atmosphere (GasPack System). The different biotypes of *E. coli were* activated in Tryptic Soy Broth (TBS) and incubated aerobically at 37°C for 18 hours. Before the experiments, the strains were subcultivated at least three times. For the spot agar test (a test known to experts in the field) the following procedure was carried out. In practice, 10 microlitres of a culture of the above-mentioned strains of lactobacilli grown at 37°C overnight (culture broth + medium) were placed (spotted) on the surface of an agarised plate (containing the agarised TBS medium). The plate was incubated for 18 hours at 37°C to allow the strain (spot) to develop. The biotypes of *E. coli* (0.1%) were incubated in Tryptic Soy soft agar and arranged on the spots in the plates referred to above. Once the top agar had solidified, the plates were inverted and incubated in conditions of aerobiosis at 37°C for 24 hours. Each test was repeated twice. After 24 hours the plates were visually examined to observe and determine the inhibition zones. The presence of an inhibition zone of 2 mm or more around the spot of lactobacilli was considered a positive result (see figure 1 for a summary of the method used).

The same technique was used for the bifidobacteria as for the lactobacilli.

### Top agar technique:

E. coli strain streaked onto agarised Petri plate (McConkei Agar medium) incubated under aerobiosis for 24h. 1 or 2 colonies are collected and dissolved in 1 ml of sterile water until they reach a turbidity value of 0.5 on the McFarland scale (known scale cited in reference tables).

100 µl of this suspension are inoculated into 50 ml of soft-agar medium (LAPTg with 0.7% agar) at a temperature of about 45°C so as to keep it liquid.

3 ml of soft-agar are poured onto the surface of the plates previously prepared with the grown lactobacilli.

Dry and incubate at 37°C under aerobiosis for 18-20h.

Results read by measuring inhibition halo.

### Results

### A. In vitro screening of the strains of the present invention against biotypes of E.coli.

The activity of inhibiting the growth of the *E. coli* was determined using the live bacterial cultures of the strains listed in Table 1. Positive results were obtained for all the strains listed in Table 2. In particular, the strains listed in Tables 3-5 were distinguished by their strong inhibition/reduction effect on the growth of all the biotypes of *E coli* tested.

### B. Selection of strains of the present invention having an inhibitory activity against toxinogenic E. coli O157:H7.

The strains listed in Table 1 were also tested against *E. coli* O157:H7. The inhibitory activity on the growth of *E. coli* O157:H7 was determined using the live bacterial cultures of the strains listed in Table 1.

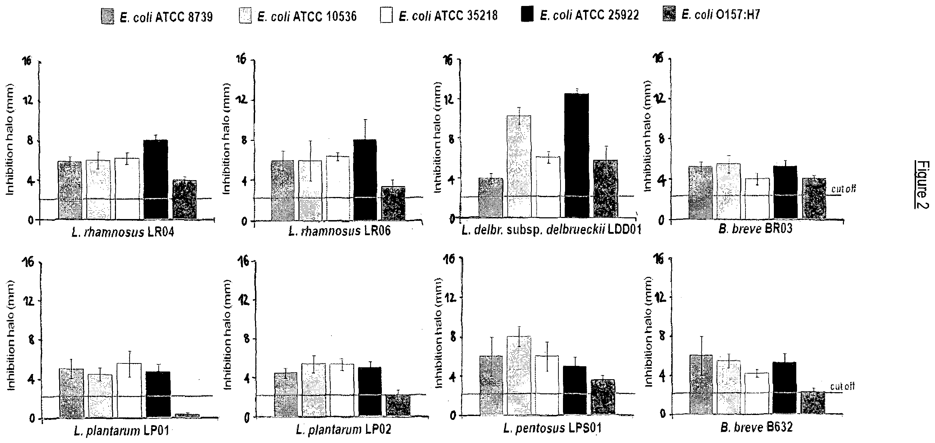

As shown in Figure 2, only 5 strains tested showed the capacity to inhibit/reduce the growth of the biotypes of *E. coli* tested, including the enterohaemorrhagic *E. coli* O157:H7 (CQ9485). In Figure 2, the results are expressed as standard deviation (SD) of the inhibition halos, calculated on three different tests and allowing a cut-off of 2 mm.

From the experimental data reported above, it can be inferred that 6 lactobacilli and 2 bifidobacteria (Table 4) show a *in vitro* inhibition/reduction activity against the growth of four strains of *E. coli* (ATCC 8739 ATCC 10536 ATCC 35218 ATCC 25922), which are responsible for the production of a wide variety of biogenic amines such as for example mercaptan, histamine, cadaverine, putrescine and tyramine by decarboxylation of the amino acids. These biogenic amines are considered responsible for dangerous systemic intoxications. Furthermore, the presence of nitrites and nitrates can form N-nitrosamines which show strong mutagenic activity that can cause some types of cancer. Finally, the experimental data reported above show that five strains of bacteria tested are active against the pathogenic strain of *E. coli* O157:H7 (CQ9485) which is capable of producing one or more of the Shiga toxins that are very dangerous for human health and can even lead to death.

### Experimental section

### Materials and methods: Micro-organisms and cultural conditions.

In the present study, 4 different species of probiotic bacteria were used, belonging to the genus *Lactobacillus* (Tables 6 and 7). All the bacteria were cultivated in Man, Rogosa and Sharpe (MRS) medium, (marketed by the company Becton Dickinson, Milan, Italy) at 37°C for 18 hours, in conditions of anaerobiosis (GasPak, Becton Dickinson, Milan, Italy). The following 4 biotypes of *E. coli* were used as target strains: *E. coli* ATCC 8739, *E. coli* ATCC 10536, *E. coli* ATCC 35218 and *E. coli* ATCC 25922, purchased from the American Type Culture Collection (ATCC, Rockville, Maryland, USA). The enterohaemorrhagic strain *E. coli* O157:H7 (CQ 9485) was offered by the Gastroenterology Department, Ospedale Maggiore della Carità, Novara, Italy. *Listeria monocytogenes* ATCC 19112 was purchased from the American Type Culture Collection; *Enterococcus* sp. and *Klebsiella* sp. were isolated from samples of faeces of neonates affected by colic (University of Bologna, Italy). All the biotypes of *E. coli*, *Enterococcus* sp. and *Klebsiella* sp were cultivated in Tryptone Soy Broth (TSB) medium (Biotest, Rockaway, NJ, USA) and incubated aerobically at 37°C for 18 hours. *Listeria monocytogenes* was cultivated in Fraser medium (Oxoid, Hampshire, United Kingdom) and incubated aerobically at 37°C for 18 hours. All the bacterial strains were transplanted at least three times before being used in the inhibition experiments.

### Fermentative profile using API 50 CHL

The species of probiotic bacteria belonging to the genus *Lactobacillus* were cultivated for 18 hours at 37°C, washed twice in sterile water and centrifuged at 6000 rpm for 5 minutes. After resuspension in water, in a test tube containing 5 ml of water a quantity of sample was added such as to reach the opacity value 2 on the McFarland scale. A quantity of sample equal to double the quantity used previously was added to 10 ml of API 50 CHL medium (API systems, BioMerieux). 200 µl of the suspension were transferred to each well of the API 50 CH gallery. All the wells were recovered with paraffin oil to ensure anaerobiosis. The galleries were moistened and incubated at 37°C. Changes in the starting colour (purple) were checked after 1 and 2 days of incubation.

### Primers and species-specific PCR assay

The genomic DNA of each strain of *Lactobacillus* is extracted with the MicroLysis™ kit (Labogen, Rho, Italy). Specifically, 2 µl of each bacterial culture is added to the lysis buffer and lysis is performed with the Gene Amp PCR System 9700 thermal cycler (Perkin-Elmer, Monza, Italy) following the manufacturer's instructions.

For the strains of *L. plantarum* to *L. pentosus,* the primers used for the PCR reaction were designed on the *rec*A gene, while for the strain of *L. rhamnosus,* the intergenic region comprised between 16S and 23S of the ribosomal RNA (Table 6) was used. All the primers were synthesised by the firm Sigma-Aldrich.

For the amplification, a mix (25 µl) was made up with 2x GoTaq Green Master Mix (Promega), 1 µl of lysed DNA and the set of species-specific primers (0.30 mM). The PCR cycles were performed with the Gene Amp PCR System 9700 thermal cycler. The amplification programs are shown in Table 6.

### PFGE

The strains of *Lactobacillus* are cultivated in MRS medium until an optical density at 600 nm (OD₆₀₀) is reached of 0.8. After washing in the buffer solution of 10 mM Tris, 20 mM NaCl, 50 mM EDTA, pH 7.2, the cells are resuspended in 300 µl of the same buffer solution. After the addition of 300 µl of agarose 2% (Bio-Rad), the samples are poured into the moulds.

The plugs are incubated for 18 hours at 37°C in the lysis buffer solution (6 mM Tris, 1 M NaCl, 100 mM EDTA, 1% sarcosyl, 0.2% deoxycholate, pH 7.6), with the addition of 2.5 mg/ml of lysozyme (Sigma) and 4 U/ml of mutanolysin (Sigma). Treatment with Proteinase K (1 mg/ml) was performed in buffer solution 100 mM EDTA, 1% sarcosyl, 0.2% deoxycholate at pH 8.0 for 24 h at 50°C. The plugs are washed 4 times with the buffer solution 20 mM Tris, 50 mM EDTA at pH 8.0, adding in the first 2 washes 1 mM phenylmethylsulfonyl fluoride (Sigma). Before digestion with the restriction enzyme, the plugs are washed twice with the TE buffer and then equilibrated for 1 hour in the appropriate restriction enzyme buffer. The digestions with the restriction enzymes are carried out for 18 hours at 37°C for Notl and at 50°C with Sfil. The electrophoretic cycle is performed with the CHEF DR III apparatus (Bio-Rad) using a 1% agarose gel (Bio-Rad) in a 0.5 M TBE buffer.

The cycle is set with the following parameters: Notl (switch time: 1-11 s, voltage 6 V/cm, buffer temperature 14°C, cycle time 27 h); Sfil (switch time: 1-15 s, voltage 5 V/cm, buffer temperature 14°C, cycle time 22 h). The agarose gel is stained with ethidium bromide (0.5 mg/ml) and viewed by means of UV light.

### Assessment of in vitro inhibitory activity

The antimicrobial activity against *E.coli* was assessed using the protocol described by Santini C. et al. ²⁶. Briefly, 10 µl of each LAB culture with optical density at 600 nm of about 1.0 are poured (drop) onto the surface of a Petri plate agarised with LAPTg medium and incubated anaerobically for 24 hours at 37°C to allow bacterial growth (spot). The target strain of *E. coli* (0.1%) is inoculated in Tryptic Soy soft agar medium and poured onto the plates containing the spots. After solidification, the plates are inverted and incubated under conditions of aerobiosis for 24 hours at 37°C.

Each experiment was performed in duplicate. The presence of a halo free of growth of the pathogen under examination (inhibition halo) was considered a positive result.

The quantification of the inhibitory activity of the probiotic in relation to different strains of *E. coli* was performed in the laboratories of the Research and Development division of the company Biolab Research, MofinAlce Group, while the quantification relating to the enterohaemorrhagic strain *E. coli* O157:H7 was carried out by the specialist staff of the private clinic "I Cedri", Fara Novarese, Italy.

Antimicrobial activity against *Listeria monocytogenes, Enterococcus* sp. and *Klebsiella* sp. was assessed using the disc diffusion method. Briefly, the LABs were cultivated in MRS medium for 24 hours at 37°C, then washed and resuspended in fresh medium. The pathogen is uniformly seeded onto the surface of Petri plate containing the agarised medium specific for the pathogenic species under examination. Different paper discs are positioned on the surface of the plate with the pathogen and 100 µl of LAB are then absorbed onto each disc. The plates are incubated at 37°C for 24 hours.

The presence of a halo free of growth of the pathogen under examination (inhibition halo) was considered a positive result. The results are expressed as inhibition halos (mm). The threshold for determining the significance of the result is 2 mm.

### Results

### In vitro inhibitory activity of probiotic strains against pathogenic bacteria

The antimicrobial activity of some strains of lactobacilli against pathogenic bacteria is multi-factorial and includes the production of hydrogen peroxide, lactic acid, known molecules such as bacteriocins and unknown molecules stable to heat.

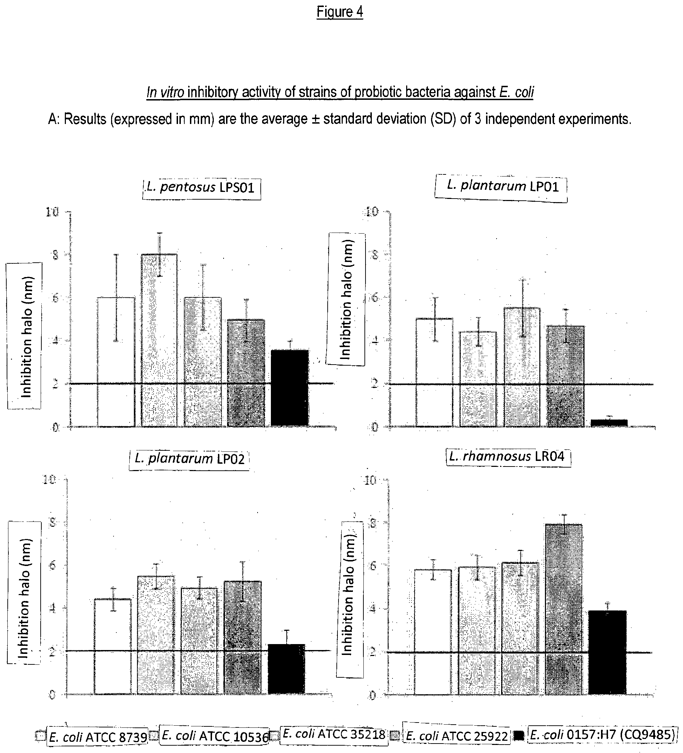

As shown in Figure 4, the results obtained by the spot method on agar and live cells highlighted the fact that all the bacteria tested show inhibitory activity in relation to the four strains of *E. coli.* The probiotic strains which display the greatest inhibitory activity are *L. rhamnosus* LR04 and *L. pentosus* LPS01. The data obtained using as target the enterohaemorrhagic strain *Escherichia coli* serotype O157:H7, too, showed the best inhibitory activity in the presence of *L. rhamnosus* LR04 and *L. pentosus* LPS01 (figure 4).

To better characterise the probiotic strains under examination, the analysis was widened to other pathogenic species which strike the human gastro-intestinal tract, using the disc diffusion method. The strain *L. plantarum* LP01 unexpectedly showed the best inhibitory capacity against all the enteropathogenic bacteria tested, in particular against *Lisferia monocytogenes.*

**Table 6**

| Bacterial strain | name | sequence (5'-3') | cycle | reference |
|---|---|---|---|---|
| *L. rhamnosus* | RHA | GCGATGCGAATTTCTATTATT | 5'95°C, (30" 94°C, 30" 58°C, 30" 72°C)x30, 7' 72°C | Appl. And Environ. Microb. 66 (2000) 297-303 |
| | PRI | CAGACTGAAAGTCTGACGGT | | |
| *L. plantarum* | PLAN-f | CCGTTTATGCGGAACACCTA | 3'94°C, (30" 94°C, 10" 56°C, 30" | Appl. And Environ. Microb. 67 (2001) |
| | P-REV | TCGGGATTACCAAACATCAC | | |
| | | | 72°C)x30, 7' 72°C | 3450-3454 |
| *L. pentosus* | PENT-f | CAGTGGCGCGGTTGATATC | 3'94°C, (30" 94°C, 10" 56°C, 30" 72°C)x30, 7' 72°C | Appl. And Environ. |
| | P-REV | TCGGGATTACCAAACATCAC | | Microb. 67 (2001) |
| | | | | 3450-3454 |

**Table 7**

| Strain | Code/Filing no. deposito |
|---|---|
| L. pentosus | LPS01 |
| L. plantarum | LP01 |
| L. plantarum | LP02 |
| L. rhamnosus | LR04 |

## Claims

1. A pharmaceutical or dietary composition or a supplement or a medical device comprising or, alternatively, consisting of at least one bacterial strain belonging to one or more species selected from the group comprising or, alternatively, consisting of *Lactobacillus plantarum* and *Lactobacillus rhamnosus* which is capable of producing bacteriocins and/or metabolites and/or hydrogen peroxide (oxygenated water) for use in the preventive and/or curative treatment of infections and/or pathologies connected with pathogens belonging to the species *E. coli*, *Listeria monocytogenes and Enterococcus sp.,* wherein said strains of bacteria are selected from the group consisting of:
- *L. rhamnosus* LR04 DSM 16605
- *L. plantarum* LP01 LMG P-21021
- *L. plantarum* LP02 LMG P-21020.

2. The composition according to claim 1, wherein said strains of bacteria have an activity against the pathogen *E. coli*; and wherein said pathogen is preferably selected from among the serotype *E. coli* O157:H7 and *E. coli* O104:H4; or from the group comprising *E.coli* ATCC 8739, *E.coli* ATCC 10536, *E.coli* ATCC 35218 and *E.coli* ATCC 25922.

3. The composition according to claim 1, wherein said strains of bacteria have an activity against the pathogen *Listeria monocytogenes* strain ATCC 19112.

4. The composition according to claim 1, wherein said composition comprises or, alternatively, consists of two or three or four or five or six strains belonging to one or more species.

5. The composition according to claim 4, wherein the bacterial strain is *L. rhamnosus* LR04 DSM 16605 for use in the preventive and/or curative treatment of infections and/or pathologies connected with pathogens belonging to the species *E. coli* serotype O157:H7 or *E*. *coli* O104:H4.

6. The composition according to claim 1 or 4, wherein the bacterial strain is *L. plantarum* LP01 LMG P-21021 for use in the preventive and/or curative treatment of infections and/or pathologies connected with pathogens belonging to the species *Listeria monocytogenes.*

7. The composition according to any one of claims 1-6, wherein said composition furthermore comprises acetylcysteine.

8. The composition according to any one of claims 1-7, wherein said composition furthermore comprises lysozyme.

9. The composition according to any one of claims 1-8, wherein said composition furthermore comprises lysozyme microencapsulated in a lipid matrix, preferably of natural origin, having a melting point comprised from 30°C to 80°C; preferably from 40°C to 70°C; even more preferably from 50°C to 60°C.

10. The composition according to any one of claims 1-9, wherein said composition furthermore comprises acetylcysteine and lysozyme microencapsulated in a lipid matrix, preferably of natural origin, having a melting point comprised from 30°C to 80°C; preferably from 40°C to 70°C; even more preferably from 50°C to 60°C.

11. The composition according to any one of claims 1-10, wherein said composition furthermore comprises at least one prebiotic fibre and/or carbohydrates with bifidogenic action.

## Patentansprüche

1. Pharmazeutische oder diätetische Zusammensetzung oder Ergänzungsmittel oder medizinische Vorrichtung umfassend bzw. bestehend aus mindestens einem Bakterienstamm einer oder mehrerer Arten aus der Gruppe, die *Lactobacillus plantarum* und *Lactobacillus rhamnosus* umfasst bzw. daraus besteht, der Bacteriocine und/oder Stoffwechselprodukte und/oder Wasserstoffperoxid (sauerstoffbeladenes Wasser) produzieren kann, zur Verwendung bei vorbeugenden und/oder heilenden Behandlungen von Infektionen und/oder Pathologien, die im Zusammenhang mit Krankheitserregern stehen, die zu den Arten *E. coli, Listeria monocytogenes* und *Enterococcus sp.* gehören, wobei die Bakterienstämme ausgewählt werden aus der Gruppe bestehend aus:
- *L. rhamnosus* LR04 DSM 16605
- *L. plantarum* LP01 LMG P-21021
- *L. plantarum* LP02 LMG P-21020.

2. Zusammensetzung nach Anspruch 1, wobei die Bakterienstämme eine Aktivität gegen den Krankheitserreger *E. coli* haben; und wobei der Krankheitserreger vorzugsweise ausgewählt wird aus dem Serotyp *E. coli* O157:H7 und *E. coli* O104:H4; oder aus der Gruppe umfassend *E. coli* ATCC 8739, *E. coli* ATCC 10536, *E. coli* ATCC 35218 oder *E. coli* ATCC 25922.

3. Zusammensetzung nach Anspruch 1, wobei die Bakterienstämme eine Aktivität gegen den Krankheitserreger des Stammes *Listeria monocytogenes* ATCC 19112 haben.

4. Zusammensetzung nach Anspruch 1, wobei die Zusammensetzung zwei oder drei oder vier oder fünf oder sechs Stämme einer oder mehrerer Arten umfasst bzw. aus zwei oder drei oder vier oder fünf oder sechs Stämmen einer oder mehrerer Arten besteht.

5. Zusammensetzung nach Anspruch 4, wobei der Bakterienstamm der Stamm *L. rhamnosus* LR04 DSM 16605 ist, zur Verwendung bei vorbeugenden und/oder heilenden Behandlungen von Infektionen und/oder Pathologien, die im Zusammenhang mit Krankheitserregern stehen, die zu den Arten *E. coli* Serotyp O157:H7 oder *E. coli* O104:H4 gehören.

6. Zusammensetzung nach Anspruch 1 oder 4, wobei der Bakterienstamm der Stamm *L. plantarum* LP01 LMG P-21021 ist, zur Verwendung bei vorbeugenden und/oder heilenden Behandlungen von Infektionen und/oder Krankheiten, die im Zusammenhang mit Krankheitserregern stehen, die zu der Art *Listeria monocytogenes* gehören.

7. Zusammensetzung nach einem der Ansprüche 1 bis 6, wobei die Zusammensetzung außerdem Acetylcystein umfasst.

8. Zusammensetzung nach einem der Ansprüche 1 bis 7, wobei die Zusammensetzung außerdem Lysozym umfasst.

9. Zusammensetzung nach einem der Ansprüche 1 bis 8, wobei die Zusammensetzung außerdem Lysozym umfasst, das in einer Lipidmatrix, vorzugsweise natürlichen Ursprungs, mit einem Schmelzpunkt im Bereich von 30°C bis 80°C; vorzugsweise 40°C bis 70°C; noch stärker bevorzugt 50°C bis 60°C; mikroverkapselt ist.

10. Zusammensetzung nach einem der Ansprüche 1 bis 9, wobei die Zusammensetzung außerdem Acetylcystein und Lysozym umfasst, die in einer Lipidmatrix, vorzugsweise natürlichen Ursprungs, mit einem Schmelzpunkt im Bereich von 30°C bis 80°C; vorzugsweise 40°C bis 70°C; stärker bevorzugt 50°C bis 60°C; mikroverkapselt sind.

11. Zusammensetzung nach einem der Ansprüche 1 bis 10, wobei die Zusammensetzung außerdem mindestens eine präbiotische Faser und/oder Kohlenhydrate mit bifidogener Wirkung umfasst.

## Revendications

1. Composition pharmaceutique ou diététique ou complément ou dispositif médical comprenant ou, en variante, consistant en au moins une souche bactérienne appartenant à une ou plusieurs espèces choisies dans le groupe comprenant ou, en variante, constitué par *Lactobacillus plantarum* et *Lactobacillus rhamnosus* qui est capable de produire des bactériocines et/ou des métabolites et/ou du peroxyde d'hydrogène (eau oxygénée), pour son utilisation dans le traitement préventif et/ou curatif des infections et/ou des pathologies liées à des pathogènes appartenant aux espèces *E. coli, Listeria monocytogenes* et *Enterococcus sp.,* où lesdites souches bactériennes sont choisies dans le groupe constitué par :
- *L. rhamnosus* LR04 DSM 16605
- *L. plantarum* LP01 LMG P-21021
- *L. plantarum* LP02 LMG P-21020.

2. Composition selon la revendication 1, dans laquelle lesdites souches bactériennes ont une activité contre le pathogène *E. coli* ; et dans laquelle ledit pathogène est de préférence choisi parmi le sérotype *E. coli* O157:H7 et *E. coli* O104:H4, ou dans le groupe comprenant *E. coli* ATCC 8739, *E. coli* ATCC 10536, *E. coli* ATCC 35218 et *E. coli* ATCC 25922.

3. Composition selon la revendication 1, dans laquelle lesdites souches bactériennes ont une activité contre la souche pathogène *Listeria monocytogenes* ATCC 19112.

4. Composition selon la revendication 1, dans laquelle ladite composition comprend ou, en variante, consiste en trois ou quatre ou cinq ou six souches appartenant à une ou plusieurs espèces.

5. Composition selon la revendication 4, dans laquelle la souche bactérienne est *L. rhamnosus* LR04 DSM 16605, pour son utilisation dans le traitement préventif et/ou curatif des infections et/ou des pathologies liées à des pathogènes appartenant à l'espèce *E. coli* sérotype O157:H7 ou *E. coli* O104:H4.

6. Composition selon la revendication 1 ou 4, dans laquelle la souche bactérienne est *L. plantarum* LP01 LMG P-21021, pour son utilisation dans le traitement préventif et/ou curatif des infections et/ou des pathologies liées à des pathogènes appartenant à l'espèce *Listeria monocytogenes.*

7. Composition selon l'une quelconque des revendications 1 à 6, dans laquelle ladite composition comprend en outre une acétylcystéine.

8. Composition selon l'une quelconque des revendications 1 à 7, dans laquelle ladite composition comprend en outre un lysozyme.

9. Composition selon l'une quelconque des revendications 1 à 8, dans laquelle ladite composition comprend en outre un lysozyme microencapsulé dans une matrice lipidique, de préférence d'origine naturelle, ayant un point de fusion de 30 à 80 °C ; de préférence de 40 à 70 °C ; plus préférablement encore de 50 à 60 °C.

10. Composition selon l'une quelconque des revendications 1 à 9, dans laquelle ladite composition comprend en outre une acétylcystéine et un lysozyme microencapsulé dans une matrice lipidique, de préférence d'origine naturelle, ayant un point de fusion de 30 à 80 °C ; de préférence de 40 à 70 °C ; plus préférablement encore de 50 à 60 °C.

11. Composition selon l'une quelconque des revendications 1 à 10, dans laquelle ladite composition comprend en outre au moins une fibre prébiotique et/ou des glucides ayant une action bifidogène.
